# EUROPEAN PATENT APPLICATION

(11) **EP 4 344 673 A1**
(43) Date of publication of application: **03.04.2024**
(21) Application number: 23169972.9
(22) Date of filing: 26.04.2023
(51) Int. Cl.: A61F 2/04, A61B 17/32, A61M 25/00, A61M 27/00, A61M 29/02

(54) **IMPLANT**

(30) Priority: 29.09.2022 US 202263411241 P
(71) Applicant: Olympus Winter & Ibe GmbH, 22045 Hamburg (DE)
(72) Inventor: HORN, Martin, 22175 Hamburg (DE); BROCKMANN, Christian, 21279 Hollenstedt (DE)
(74) Representative: Hoener, Matthias

(57) **Abstract**

The invention creates an implant with which prostates of different sizes can be treated. This is achieved by allowing the length of a deployed implant (10) to be variably changed. By changing the length of a stretched wire structure (11) of the implant (10), the implant can be individually adapted to the patient's anatomy, thereby increasing the treatment success.

## Description

The invention relates to an implant according to the claims 1, 11 and 14.

Various methods or techniques are known for the treatment of the urinary tract, in particular *benign prostatic hyperplasia (BPH).* In a minimally invasive, particularly body-sparing treatment of BPH symptoms, a removable implant is temporarily placed in the urethra or in the prostatic portion of the patient's urethra. Such an implant is a wire structure made of a shape memory alloy, such as nitinol. The wire structure is inserted in a collapsed/folded state through a catheter into the urinary tract and pushed into position to unfold into its predetermined basic structure. A tongue-like retaining element or holding element, which may also be part of the implant, engages the bladder neck to fix the wire structure in place. The wire structure, which may be formed from three or four wires, is a basket structure. This basket structure expands the urethra. Due to the expansion of the wire structure against the tissue of the urethra, the tissue of the urethra is denatured in the course of a few days. This denaturation of the tissue occurs due to the ischemic pressure of the individual wires on the cells of the tissue, resulting in decreased or complete lack of blood flow. As a result, the lack of blood flow leads to a lack of oxygen in the cells and eventually to cell death. Within a few days, the tissue can be reduced to such an extent that the urinary flow almost returns to normal. After completion of this treatment, the implant can be retrieved from the urethra by means of a catheter.

In order to also treat the bladder neck during treatment, the wires of the implant protrude into the bladder. The tongue-like holding element of the implant is pulled behind the bladder neck to lock the implant inside the prostate. Now, it so happens that patients' anatomies are different and their prostates may also be enlarged to different degrees. If the prostate is relatively short, there is a risk that the implant or wires will be placed too deep towards the external sphincter within the bladder neck. This may cause temporary or even permanent incontinence in the patient.

On this basis, the invention is based on the problem of creating an implant with which prostates of different sizes can be treated.

A solution to this problem is described by the features of claim 1. Accordingly, it is provided that the length of the deployed implant can be variably changed. By changing the length of the unfolded wire structure, the implant can be individually adapted to the patient's anatomy, thereby increasing the success of the treatment. Side effects, such as incontinence of the patient, can be avoided by the individual length adjustment. In order to be able to specifically adjust the length of the implant or the wire structure, the length of the enlarged prostate is measured before the patient is treated. This can be done, for example, by an imaging procedure, such as ultrasound, or some other procedure.

Preferably, the invention provides that a sleeve is slidable over a proximal end region of the at least two wires of the wire structure, said sleeve being initially freely slidable over the proximal end region of the wires. The at least two or three wires form the wire structure, which in turn forms the main component of the implant. At a distal end, the wires are close together or in contact and interconnected. The proximal ends of the wires are also connected to each other and, if necessary, connected to a holding means to be pushed through the catheter into the urethra and to pull the structure out of the urethra after treatment. Immediately after the initially folded wire structure is pushed out of the catheter, the wires, which are parallel in the folded state, expand into their predefined basket structure. This transition into the predefined basket shape is achieved by the shape memory material used for the wires. After completion of the treatment, the wire structure is restored to the collapsed structure by applying a small axial tensile force to the proximal end of the wires. The invention provides for the sleeve to be associated with the proximal end portion of the collapsed wires, which may range from a few millimeters to centimeters in length. In this case, the hollow cylindrical sleeve comprises the two or three wires. Depending on the pre-measured size of the prostate, the sleeve can be slid to a specific position on the wires.

Further, it is particularly provided that the sleeve is fixedly connectable to the at least two or three wires at the defined position, wherein the fixed connection prevents relative movement between the wires and the sleeve. The sleeve prevents the wire structure from unfolding into the predefined shape outside the catheter. In fact, only the area of the wires from the distal end to the sleeve unfolds; that is, with a shortened length. Accordingly, the further the sleeve is positioned in the distal direction on the wires, the less the wires unfold and the less the implant adversely affects the proximal area near the external sphincter. The position of the holding element remains unaffected by this, so that the wires or the wire structure always protrude the same distance into the bladder, which is essential for the success of the therapy.

A particular embodiment of the invention may in particular provide that the sleeve is a screw sleeve, a crimp sleeve, a click sleeve, a snap-in sleeve or a sleeve with an internal passage whose diameter is just equal to the sum of the diameters of the wires. In these embodiments of the sleeve, it is essential that the sleeve can be fixed to the wires in a simple manner and in such a way that it retains its position even in the face of light mechanical tensile forces. The sleeve can be made in one piece or in two or more pieces.

In particular, it is conceivable that the screw sleeve can be assembled from a first part and a second part, both parts being of hollow cylindrical design and the wires being guided through the interior of this hollow cylinder. The first part has an female thread and the second part has a sleeve with an male thread, so that the two parts can be screwed together and the wires move through the two parts. A ring made of an elastic material is positioned between the two parts, which expands when the two parts are screwed together to encompass the wires for a tight connection between the sleeve and the wires. The click sleeve and snap-in sleeve designs are technically similar to the screw sleeve. The two aforementioned types of sleeves also consist of two parts that can be joined together by applying a mechanical force, whereby an elastic ring between the two parts is deformed during the joining process, thereby achieving a fixed connection between the sleeve and the wires. This connection between the two parts is reversible, so that the sleeve can be removed or repositioned after the treatment, but also during the treatment. For example, it is conceivable for the application that by repositioning the sleeve during treatment, the length of the implant can be changed to respond to the progress of the treatment. This allows the treatment of the individual to be made even more efficient.

When using a crimp sleeve, the sleeve is fixed to the wires with a crimping tool. The use of a crimp sleeve has the advantage that it is particularly resistant to axial forces acting on the connection between the sleeve and the wires. Finally, another alternative provides that the inner diameter of the hollow cylindrical sleeve is matched to the sum of the diameters of the wires and the friction of the inner wall of the sleeve on the wires is sufficient to guarantee the fixation of the sleeve at a certain position on the wires. In this case, an increased amount of force is required for positioning the sleeve in order to bring the sleeve into position on the wires against the frictional forces. Advantageously, however, in this embodiment the sleeve turns out to be constructively very simple. Furthermore, the sleeve can be fastened without much effort. In addition, this type of sleeve can be manufactured with a particularly small outer diameter.

According to the invention, it can be provided that the sleeve can be fixed on the wires outside the person before the treatment or inside the person during the treatment. Provided that the size of the prostate is already known prior to treatment, the position of the sleeve can be specifically positioned and fixed outside the patient prior to treatment so that the implant opens within the urethra with the predefined length. Similarly, it is conceivable that during treatment it is determined where to place the sleeve so that the prostate can be treated particularly efficiently. For this purpose, it is conceivable that further imaging instruments are inserted into the patient to determine the position. Furthermore, it is conceivable that another tool or forceps is inserted into the patient to fix the sleeve on the wires. In this embodiment, it is also conceivable that the position of the sleeve on the wires is readjusted during treatment in order to be able to react efficiently to possible treatment successes already during treatment.

The present invention may provide that the outer diameter of the sheath is less than 3 mm. This diameter is essentially dependent on the catheter or implant used. Where possible, it is also conceivable that sleeves with a smaller outer diameter, such as 2 mm or 1 mm, may be used.

Another preferred embodiment of the invention may provide that at least one of the wires or a holding element of the implant has markings by means of which the length of the implant or position of the sleeve can be adjusted. Thus, it may be helpful to the surgeon if, when the size of the prostate is determined, he only has to push the sleeve to a certain mark in order to fix the sleeve there with the wires. Depending on the position of the sleeve on the wires, the wire structure unfolds with a certain length. The relationship between the size of the prostate and the marking can be seen, for example, in a previously created table.

A further solution to the above-mentioned problem is described by the features of claim 11. Accordingly, it is provided that the length of a retaining element or holding element can be variably changed. In this case, the length of the wires remains unchanged and only the length of the tongue-like holding element is changed. The clinical effect is the same as for changing the length of the switched-off wire structure. The essential point here is that the three wires still protrude into the bladder. Thereby, in this embodiment example, the tongue-like holding element can be varied in length and fixed by means of a sleeve described above. According to this embodiment example, the position of the holding element in the urethra is adjusted in order to optimally position the implant even with prostates of different sizes.

Alternatively, it is also conceivable that the holding element has a segment-like design and can be shortened at several predetermined breaking points. Depending on the need or size of the prostate, individual distal elements of the holding element can be cut off for treatment. A sleeve, as in the previously described embodiments, is not necessary.

A solution to the above task is further described by the features of claim 14. Accordingly, it is provided that the wires and the tongue-like retaining element or holding element are variable in their lengths. Thereby, the lengths of the wires and the holding element can be varied as previously described. By this specific variation of the lengths of the wires and the holding element, it is possible to react particularly efficiently and effectively to almost any patient anatomy and size of the prostate; respectively, depending on the anatomical conditions, it is possible to choose between an adjustment of the wire structure size and the length of the holding element. Similarly, the two aforementioned components can also be adjusted in size during treatment, as described above, so that the patient's treatment results in the best possible success.

A preferred embodiment of the present invention is explained in more detail below with reference to the drawing. In this show:
- Fig. 1: a representation of a deployed implant,
- Fig. 2: a representation of the implant in the folded state,
- Fig. 3: a representation of the implant in the deployed state,
- Fig. 4: a representation of the implant in a shortened, folded state, and
- Fig. 5: a sectional view of an embodiment of a sleeve.

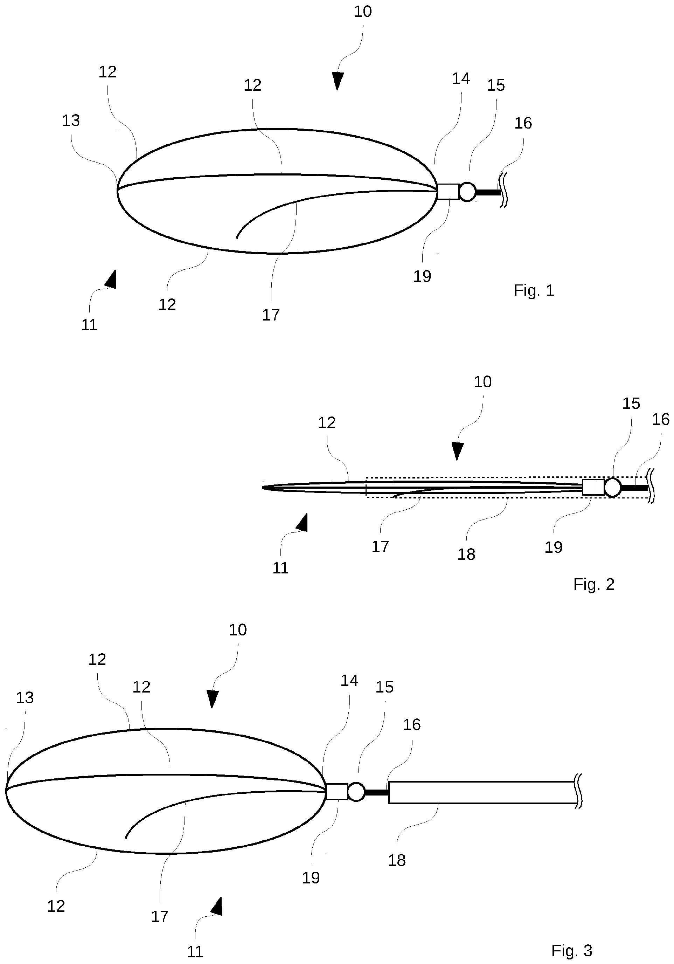

Fig. 1 shows a possible embodiment of an implant 10. It should be expressly noted that this example is only one of many conceivable embodiments.

The embodiment of the stretched implant 10 shown in Fig. 1 has a wire structure 11 with three wires 12. However, embodiments with two or four or more wires 12 are also conceivable. However, it has been shown that three wires 12 are particularly suitable for manipulating the tissue of the urethra. Advantageously, these wires 12 are wires made of stainless steel, a spring steel or a material with a shape memory. Alternatively, it is also conceivable that the wires 12 are formed as plastic rods. Thus, in a particularly advantageous embodiment example, it is provided that the plastic is biodegradable. The implant 10 thus at least partially dissolves in the body after some time, so that further intervention to recover the implant 10 is not necessary.

The wires 12 are connected to each other at their distal ends 13. The opposite proximal ends 14 of the wires 12 are brought together in a common connecting body 15. This connecting body 15 is shown in the figures as a sphere, but can also have any other shape. A holding means 16 is arranged proximally on the connecting body 15. This holdung means 16, which may be in the form of a thread, a flexible wire or a pin, is used in particular for placing the implant 14 in the urethra and for withdrawing the implant 10 from the urethra after completion of the treatment. Accordingly, the holding means 16 is guided out of the body during the treatment.

Fig. 1 also shows a highly schematized tongue-like holding element 17. This holding element 17 engages the tissue after the implant 10 has been correctly positioned in the urethra and serves to fix the implant 10 in the area to be treated. This holding element 17 can preferably be made of the same material as the wires 12 and is also connected to the holding means 16. Due to the tongue-like shape of the holding element 17, it acts like an anchor for the implant 10. By briefly pulling back after positioning the implant 10 in the proximal direction, the holding element 17 is fixed in the tissue.

In the embodiment shown in Fig. 1, the sleeve 19 according to the invention is also shown schematically. This sleeve is a hollow cylinder which is pushed over the proximal ends 14 or over a proximal region of the wires 12. Initially, i.e. before treatment, this sleeve 19 is freely displaceable over the wires 12. For the adjustment of a certain length of the implant 10 or the wire structure 11, the sleeve 19 can be moved back and forth along the folded wires 12 and fixed at an appropriate position. The distance between the distal end 13 and the sleeve 19 then defines the length of the stretched implant 10. In the embodiment example shown in Fig. 1, the sleeve 19 is positioned directly in front of the connecting body 15, i.e., the sleeve 19 encloses the proximal ends 14 of the wires 12. This positioning of the sleeve 19 stretches the wire structure 11 to its maximum length. The length of the implant 10 or wire structure 11 can be determined as a function of the size of the prostate. This size or length of the prostate is determined prior to treatment, so that the optimum length of the wire structure 11 can already be set before the implant 10 is inserted into the patient's body by moving and fixing the sleeve on the wires 12.

For the treatment of BPH syndrome, the implant 10 is first inserted in the folded state through a tube-like catheter 18, as schematically shown in Fig. 2, into the patient's urethra. In this process, the sleeve 19 is located at the proximal end 14 of the wires 12 in the embodiment example according to Figs. 1 and 2, so that the wire structure 11 has a maximum length. Once the catheter 18 has been brought to the correct position within the urethra, the implant 10 is pushed out of the catheter 18 and, if necessary, at the same time the catheter 18 is pulled out of the urethra in the proximal direction. Due to the chosen material, the wires 12 stretch outside the catheter 18 to form the wire structure 11 shown in Fig. 1, namely up to the sleeve 19. Alternatively, it is also conceivable that the wires 12 are stretched to form the wire structure 11 by an axial pull on the holding means 16. Should the implant 10 move away from the optimal position during the clamping of the wire structure 11, the implant 10 can be moved back into the correct position.

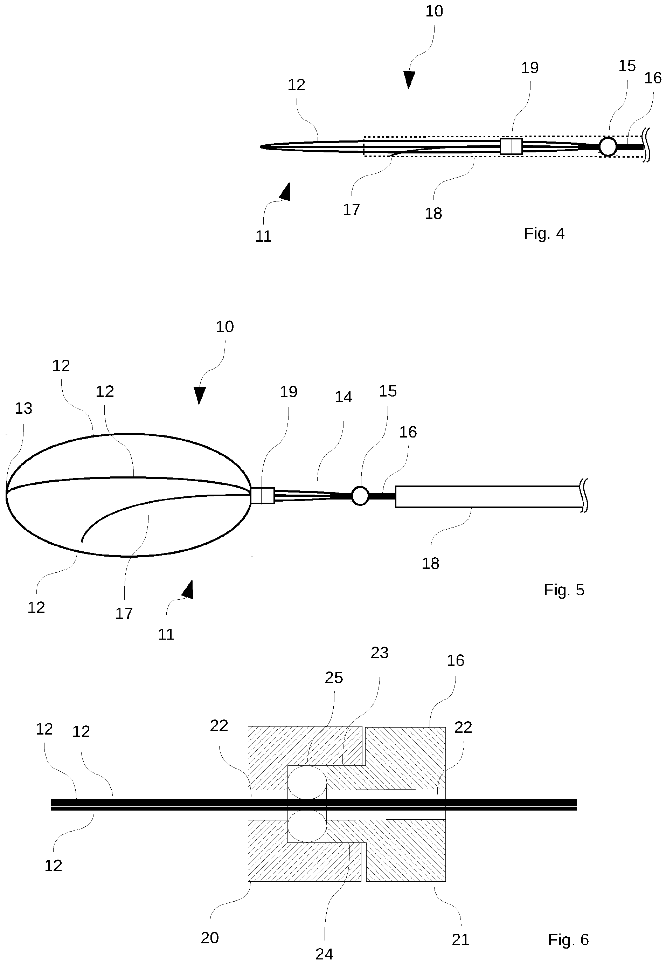

As soon as the wire structure 11 has reached the optimal shape or the maximum spread to treat the tissue and the holding element 17 has been anchored in the tissue, the catheter 18 is pulled out of the urethra in the proximal direction. The implant 10 remains in the predetermined position thanks to the anchoring by the holding element 17, with the holding means 16 being guided out of the urethra (Fig 3). After completion of the treatment, the catheter 18 is reinserted into the urethra via the holding means 16 and the stretched wire structure 11 is retracted into the catheter 18 by the holding means 16, with the wires 12 being contracted around the circumference of the catheter 18. In the collapsed state, the implant 10 can be pulled out of the urethra again in the catheter 18. For the treatment of a smaller prostate, the length of the implant 10 can be varied. It can be seen from Fig. 4 that the sleeve 19 is displaced in the distal direction compared to the embodiment example according to Figs. 1 to 3. In this case, the sleeve 19 is not only displaced in the distal direction, but is also fixed at this position. This positioning of the sleeve 19 and the fixation already take place outside the catheter 18 depending on the determined size of the prostate. Apart from the position of the sleeve 19 on the wires 12, no changes have been made compared to the embodiment example according to Figs. 1 to 3. When the implant 10 is brought out of the catheter 18, the wires 12 stretch open in the same way as previously described, but only as far as the sleeve 19. The sleeve 19 prevents the complete deployment of the implant 10. Accordingly, according to the embodiment example of Fig. 5, the implant 10 is shortened. This shortened length of the implant 10 is adapted to the size of the prostate to be treated. By this adjustment of the length of the wire structure 11, no other tissues or muscles or the like are unintentionally damaged during the treatment. Rather, this adjusted length applies pressure precisely to the area to be treated.

Just like the length of the wires 12, the length of the tongue-like holding element 17 can also be varied. In this case, the holding element 17 can be guided equally through the sleeve 19. Embodiments are conceivable in which only the holding element 17 is guided through the sleeve 19 and the wires 12 are not affected in their stretched length, or that the wires 12 are guided through the sleeve 19 together with the holding element 17. Provided that only the holding element 17 is guided through the sleeve 19 and its length is shortened, the length of the implant 10 is not changed, but the position at which the implant 10 is fixed within the bladder neck is changed. In doing so, the implant 14 may protrude into the bladder, but will not make contact with the external sphincter.

In order for the implant 10 to maintain its set length during treatment, it is of paramount importance that the sleeve 19 can be firmly connected to the wires 12 and/or to the holding element 17. Only through this firm connection do the wires 12 tighten distally from the sleeve 19, and without the sleeve 19 being displaced in the proximal direction in the process. Various embodiments of the sleeve 19 are provided for this fixation of the sleeve to the wires 12. In Fig. 6, a possible embodiment example is shown. According to this, the sleeve comprises a first part 20 and a second part 21. These two parts 20, 21 are of hollow-cylindrical design and can be screwed into one another. Furthermore, both parts 20, 21 have a passage 22 through which the wires 12, exemplified in the embodiment example according to Fig. 6, and/or the holding element 17 can be passed. The first part 20 has an female thread 23 that corresponds to an male thread 24 of the second part 21. Thereby, the male thread 24 of the second part 21 is arranged at an area with a reduced diameter that can be screwed into the first part 20. An elastic ring 25 is also positioned between the first part 20 and the second part 21. When the first part 20 is screwed to the second part 21, this elastic ring 25 is compressed so that the free passage 22 is reduced. This deformation of the ring 25 fixes wires 12 within the sleeve 19 so that relative movement between the wires 12 and the sleeve 19 is prevented.

It is envisaged that the screwing of the sleeve 19 is carried out outside the catheter 18 before the treatment. It is conceivable that there are markings on the wires 12 to give the surgeon an indication of where to place the sleeve. The markings can be used to set a specific length of the implant. Similarly, it is also conceivable that the sleeve 19 is screwed inside the catheter. For example, even after the implant 10 has been placed in the urethra during treatment, the screw connection can still be opened, the sleeve can be moved on the wires 12 and screwed again, so that the length of the implant 10 can be actively changed during treatment. For this purpose, it is conceivable that the sleeve 19 is gripped and actuated within the urethra using a special tool.

An alternative embodiment of the sleeve can provide for it to be designed as a click or latching sleeve. In this case, two parts are not screwed together, as previously described with reference to Fig. 6, but are clicked or latched together. A further embodiment of the sleeve may provide that it is designed as a crimp sleeve. In this case, the crimp sleeve is guided over the wires 12 before being treated outside the body and crimped with a crimping tool. A crimp connection of this type offers particularly reliable fixing of the sleeve and is also particularly inexpensive to manufacture.

Regardless of its embodiment, the sleeve 19 can be made of a metal, plastic or ceramic. The diameter of the sleeve 19 or of the two parts 20, 21 is essentially based on the internal diameter of the catheter 18, but is generally less than 3 mm. Embodiments are conceivable in which the diameter of the sleeve can also be 1 mm to 2 mm.

### List of reference signs:

- 10: Implant
- 11: Wire structure
- 12: Wire
- 13: Distal end
- 14: Proximal end
- 15: Connection body
- 16: Holding means
- 17: Holding element
- 18: Catheter
- 19: Sleeve
- 20: First part
- 21: Second part
- 22: Passage
- 23: Female thread
- 24: Male thread
- 25: Ring

## Claims

1. Removable implant (10) for treating a urinary tract of a person by applying a local ischemic pressure to the tissue of urinary organs, in particular the urethra, through a wire structure (11) having at least two wires (12), wherein the implant (10) is insertable into the urethra in a folded state with a distal end (13) first and unfolds in the urethra to the wire structure (11) for treatment of the tissue, **characterized in that** the length of the unfolded implant (10) is variably changeable.

2. Removable implant (10) according to claim 1, **characterized in that** a sleeve (19) is slidable over a proximal end region of the at least two wires (12), said sleeve (19) being freely slidable over the proximal end region of the wires (12).

3. Removable implant (10) according to claim 1 or 2, **characterized in that** the sleeve (19) is fixedly connectable to the at least two wires (12), the fixed connection preventing relative movement between the wires (12) and the sleeve (19).

4. removable implant (10) according to any one of the preceding claims, **characterized in that** the sleeve (19) is a screw sleeve, a crimp sleeve, a click sleeve, a snap-in sleeve or a sleeve (19) with an internal passage (22) whose diameter is just equal to the sum of the diameters of the wires (12).

5. Removable implant (10) according to any one of the preceding claims, **characterized in that** the positioning of the sleeve (19) on the at least two wires (12) adjusts the length of the implant (10), wherein the fixing of the sleeve (19) on the wires (12) fixes the length of the unfolding portion of the wires (12).

6. Removable implant (10) according to claim 4, **characterized in that** the screw sleeve (19) can be assembled from a first part (20) and a second part (21), the first part (20) having an female thread (23) and the second part (21) having a sleeve with an male thread (24), the second part (21) being screwable to the first part (20) and an elastic ring (25) being clampable between the two parts (20, 21).

7. Removable implant (10) according to any of the preceding claims, **characterized in that** the sleeve (19) is fixable to the wires (12) outside the person before treatment or inside the person during treatment.

8. Removable implant (10) according to any one of the preceding claims, **characterized in that** the sleeve (19) is releasably attachable to the wires (12).

9. removable implant (10) according to any of the preceding claims, **characterized in that** the sleeve (19) has an outer diameter of less than 3 mm.

10. Removable implant (10) according to any of the preceding claims, **characterized in that** at least one of the wires (12) or a holding means (16) of the implant (10) has markings by means of which the length of the implant (10) or the position of the sleeve (19) can be adjusted.

11. A removable implant (10) for treating a urinary tract of a person by applying a local ischemic pressure to the tissue of urinary organs, in particular the urethra, through a wire structure (11) having at least two wires (12), wherein the implant (10) is insertable in a folded state with a distal end (13) first into the urethra and unfolds in the urethra to the wire structure (11) for treatment of the tissue, and with a holding element (17) by which the position of the implant (10) within the urinary tract is fixable, **characterized in that** the length of the holding element (17) is variably changeable.

12. Removable implant (10) according to claim 11, **characterized in that** a sleeve (19) according to at least one of the claims 2 to 10 is slidable over a proximal end region of the retaining element (17).

13. removable implant (10) according to claim 11, **characterized in that** the retaining element (17) has predetermined breaking points at a distal end, by means of which the length of the retaining element (17) can be selectively changed.

14. A removable implant (10) for treating a urinary tract of a person by applying a local ischemic pressure to the tissue of urinary organs, in particular the urethra, through a wire structure (11) having at least two wires (12), wherein the implant (10) is insertable into the urethra in a folded state with a distal end (13) in front and unfolds in the urethra to the wire structure (11) for treatment of the tissue, and with a holding element (17) by which the position of the implant (10) within the urinary tract is fixable, **characterized in that** according to claims 1 to 13 the length of the implant (10) and the length of the holding element (17) are variably changeable.
